# EUROPEAN PATENT APPLICATION

(11) **EP 2 263 528 A1**
(43) Date of publication of application: **22.12.2010**
(21) Application number: 09162663.0
(22) Date of filing: 15.06.2009
(51) Int. Cl.: A61B 5/0205, A61B 5/029

(54) **Apparatus and method for determining physiologic parameters of a patient**

(71) Applicant: Pulsion Medical Systems AG, 81829 München (DE)
(72) Inventor: Dr. Gödje, Oliver, 82064, Strasslach (DE); Dr. Scheier, Jörg, 80639, München (DE)
(74) Representative: Ettmayr, Andreas

(57) **Abstract**

The apparatus comprises a patient monitor (4) reading in a pressure signal from a pressure sensor (2) measuring an arterial pressure of the patient (6) employing an arterial catheter (1). The patient monitor (4) can also read in an electrocardiography signal from an ECG device (15) The patient monitor (4) determines curve characteristics indicative for a breathing state and/or a heart rhythm state of the patient (6) and switches from a first volume responsiveness indication state to a second volume responsiveness indication state, if a first condition is met by the curve characteristics, and from the second volume responsiveness indication state to the first volume responsiveness indication state, if a second condition is met. Thus, two visually and/or audibly distinguishable indication states are provided depending on
- whether the relevant curve characteristics indicate that the patient is in an arrhythmic condition, and/or
- whether the relevant curve characteristics indicate that the patient is breathing spontaneously.

## Description

The present invention relates to an apparatus for determining physiologic parameters of a patient. In particular, the invention relates to an apparatus for determining physiologic parameters of a patient which comprises at least one pressure sensor device adapted to provide readings of a blood pressure of the patient, storage means for storing the readings as at least one pressure curve over time and an evaluation unit for determining physiologic parameters from said pressure curve.

Furthermore, the invention also relates to a method of determining physiologic parameters of a patient using an electronic apparatus, wherein a signal representing a blood pressure of the patient is read in, the readings are stored as at least one pressure curve over time, and physiologic parameters are determined from said pressure curve.

Apparatus and methods of the type initially mentioned are well-known from the prior art and widely used in bedside monitoring of critically ill patients. Beside simple applications wherein the only physiologic parameter to be determined is the blood pressure itself, a pulse contour analysis is usually performed to determine various physiologic, more particular hemodynamic, parameters from the (temporarily) stored pressure curve. EP 0 947 941 A2, for example, describes in-vivo determination of the compliance function and the cardiac output of a patient using pulse contour analysis.

Cardiac output is among the most important parameters determined in bedside monitors. By definition, cardiac output (CO) is equal to the stroke volume multiplied by the heart rate (HR). The three principal factors affecting stroke volume are preload, afterload and myocardial contractility. A common definition of preload is the volume of blood that remains in the left ventricle at the end of the diastole, i.e. the left ventricular end diastolic volume (LVEDV). Generally, preload is a reflection of the volume status of a patient (i.e. the filling state of the patient's circulatory system), but it cannot be measured directly in clinical practice. In clinical practice, afterload is considered as impedance or resistance to ventricular contraction, but in strict physiological sense is defined as the transmural pressure difference of the left ventricle during systole. Myocardial contractility is defined as the ability of the myocardial muscle to contract.

If cardiac output of a monitored patient is too low, the physician in charge will have to consider counter measures that may have to be taken. Generally, depending on the filling state of the circulatory system, and also on the individual patient, adding volume may have a significant or only little effect on cardiac output. A term frequently used, to describe this behaviour is volume responsiveness. In clinical practice often blood volume is increased and the reaction of the patient organism is observed. However, adding volume may involve the risk of creating a pulmonary edema. Therefore, there has been a considerable endeavour to determine parameters helping the physician in charge to correctly assess volume-responsiveness, i.e. to assess whether the cardiovasculary system will respond to adding fluid by an increased cardiac output (CO) and which CO will result.

Fr6d Michard and Jean-Louis Teboul, "Predicting fluid responsiveness in ICU patients", Chest 121 (2002), 2000-2008 and D.A. Reuter et. al., "Optimizing fluid therapy in mechanically ventilated patients after cardiac surgery by on-line monitoring of left ventricular stroke volume variations. Comparison with aortic systolic pressure variation. Br. J. Anaesth. 88 (2002), 124-126 disclose using the parameters stroke volume variation (SVV) and pulse pressure variation (PPV) for determining volume-responsiveness of a patient.

US 6,776,764 B2 discloses a treatment algorithm for managing hemodynamically unstable patients by means of calculated pulse pressure variation (PPV) or stroke volume variation (SVV). Depending on the patient's state, volume management, vasopressor management or inotropic and cardiac management are suggested.

However, due to the general limitations of PPV and SVV measurement, these approaches are generally limited to controlled mechanically ventilated patients and cannot be applied to spontaneously breathing patients. In the following, "mechanical ventilation" is understood as fully (pressure- or volume-) controlled mechanical ventilation without substantial spontaneous breathing efforts, whereas "spontaneous breathing" comprises spontaneous breathing without a ventilator attached to the patient, spontaneous breathing on the ventilator, and most kinds of combinations of spontaneous and mechanical ventilation modes (commonly named "assisted ventilation", as e.g. pressure support ventilation).

Other known approaches for assessing the volume responsiveness of a patient also often depend on the patient's breathing state.

Further, assessment of the volume responsiveness based on SVV and/or PPV may fail due to an arrhythmic condition of the patient.

If volume responsiveness is assessed on the basis of parameters that have been determined outside the regime of their actual applicability, there is a severe danger of wrong diagnostic conclusions which may implicate wrong therapeutic decisions up to the risk of causing patient's death.

It is therefore an object of the present invention to avoid such negative implications of inappropriate parameter determination.

According to one aspect of the present invention this object is achieved by an apparatus according to claim 1, i.e. an apparatus of the kind initially mentioned, wherein the evaluation unit of the apparatus is adapted to determine curve characteristics indicative for a breathing state and/or a heart rhythm state of the patient and is further adapted to switch from a first volume responsiveness indication state to a second volume responsiveness indication state, if a first condition is met by the curve characteristics, and to switch from said second volume responsiveness indication state to said first volume responsiveness indication state, if a second condition is met by the curve characteristics.

Thus, the present invention provides two different indication states depending on
- whether the relevant curve characteristics indicate that the patient is in an arrhythmic condition, and/or
- whether the relevant curve characteristics indicate that the patient is breathing spontaneously.

In this context, indication states are understood to be states that can be visually and/or audibly distinguished by the user of the apparatus.

Advantageous embodiments of the present invention can be configured according to any of claims 2-8.

It has been found that in some situations medical staff may - in particular in strenuous situations and under lack of time - tend to trust in parameters displayed on a patient monitor or similar apparatus without questioning whether the actual conditions allow for applying the respective parameter. The present invention now allows to warn the user about parameter values that have been (at least according to a certain probability) determined outside the regime of their applicability (or applicability of the relevant determination algorithms, respectively). According to an advantageous embodiment, the present invention may implemented in a manner even preventing a certain parameter from being displayed (or otherwise output) at all when the respective parameter has been (at least according to a certain probability) determined outside the regime of its applicability (or applicability of the relevant determination algorithm, respectively).

According to a preferred embodiment, the curve characteristics indicative for a breathing state comprise the distance between consecutive relative maxima and/or consecutive relative minima of an envelope of said pressure curve. This distance can advantageously either be a peak-to-peak distance in the two dimensional coordinate system or a distance on the abscissa (i.e. the time interval between two respective extrema). Instead of a distance between maxima or minima, respectively, a distance between consecutive extrema (e.g. the distance between a relative maximum and the relative minimum following said maximum and/or vice versa) can also be used.

In such an embodiment the first condition advantageously includes that a measure of variation of the distance between consecutive relative maxima and/or minima exceeds a respective first threshold (hereinafter first extrema interval variation threshold), and the second condition includes that the measure of variation of the distance between consecutive relative maxima and/or minima falls below a respective second threshold (hereinafter second extrema interval variation threshold).

According to a particularly advantageous embodiment of the present invention, the evaluation unit further comprises an input channel for an electrocardiography signal and the curve characteristics indicative for a heart rhythm state comprise the distance (time interval) between consecutive R-peaks of said electrocardiography signal (RR-interval).

In such an embodiment the first condition advantageously includes that a measure of variation of the distance (time interval) between consecutive R-peaks exceeds a respective first RR interval variation threshold, and the second condition advantageously includes that the measure of variation of the distance between consecutive R-peaks falls below a respective second RR interval variation threshold.

According to another preferred embodiment, the evaluation unit is further adapted to determine a volume responsiveness indicator indicative for a volume responsiveness of the patient and to output the volume responsiveness indicator.

Preferably, determination and/or output of the volume responsiveness indicator is disabled in the second volume responsiveness indication state, and determination and output of the volume responsiveness indicator are both enabled in the first volume responsiveness indication state.

Preferably, the evaluation unit is adapted to determine at least one of pulse pressure variation (PPV) and stroke volume variation (SVV) from the pressure curve, and to determine the volume responsiveness indicator from at least one of PPV and SVV.

According to an advantageous embodiment, the evaluation unit is further adapted to output an alert signal indicating whether the present volume responsiveness indication state is the first volume responsiveness indication state or the second volume responsiveness indication state.

Advantageously, suppressing means may be provided for suppressing switching between the first volume responsiveness indication state and the second volume responsiveness indication state, wherein the suppressing means can be activated and de-activated by a user.

According to another aspect of the present invention this object is achieved by a method according to claim 9, i.e. a method for determining physiologic parameters of a patient using an electronic apparatus, wherein the method includes
- reading in a signal representing a blood pressure of said patient,
- storing said readings as at least one pressure curve over time,
- determining physiologic parameters from said pressure curve, and
- determining curve characteristics indicative for a breathing state and/or a heart rhythm state of said patient.

The electronic apparatus is switched from a first volume responsiveness indication state to a second volume responsiveness indication state, if a first condition is met by the curve characteristics, and switched from the second volume responsiveness indication state to the first volume responsiveness indication state, if a second condition is met by said curve characteristics.

Advantageous embodiments of the method can be carried out according to any of claims 10-14.

In particular, the curve characteristics indicative for a breathing state may advantageously comprise the distance between consecutive relative maxima and/or consecutive relative minima of an envelope of said pressure curve. This distance can advantageously either be a peak-to-peak distance in the two dimensional coordinate system or a distance on the abscissa (i.e. the time interval between two respective extrema). Instead of a distance between maxima or minima, respectively, a distance between consecutive extrema (e.g. the distance between a relative maximum and the relative minimum following said maximum and/or vice versa) can also be used. In such an embodiment the first condition advantageously includes that a measure of variation of the distance between consecutive relative maxima and/or minima exceeds a respective first threshold (hereinafter first extrema interval variation threshold), and the second condition includes that the measure of variation of the distance between consecutive relative maxima and/or minima falls below a respective second threshold (hereinafter second extrema interval variation threshold).

The method further preferably includes the step of reading in an electrocardiography signal into the electronic apparatus. The curve characteristics may then advantageously comprise the distance between consecutive R-peaks of said electrocardiography signal (RR-interval).

In such an embodiment the first condition advantageously includes that a measure of variation of the distance (time interval) between consecutive R-peaks exceeds a respective first RR interval variation threshold, and the second condition advantageously includes that the measure of variation of the distance between consecutive R-peaks falls below a respective second RR interval variation threshold.

Preferably, the method further includes; determining a volume responsiveness indicator indicative for a volume responsiveness of the patient and outputting the volume responsiveness indicator.

Preferably, the volume responsiveness indicator is disabled in the first volume responsiveness indication state, and determination and output of said volume responsiveness indicator are both enabled in said first volume responsiveness indication state.

Preferably at least one of pulse pressure variation (PPV) and stroke volume variation (SVV) are determined from the pressure curve, and the volume responsiveness indicator is determined from at least one of PPV and SVV

According to yet another aspect of the present invention, a storage medium is provided having physically stored thereon a computer program comprising executable instructions for causing a computer to perform a method as described above.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise.

The invention will now be described in more detail. The accompanying drawings, which are schematic illustrations, serve for a better understanding of the features of the present invention.

Therein
Fig. 1 shows a basic setup of an embodiment of the apparatus according to the present invention, which is connected to an EKG device,
Fig. 2 shows a setup of an embodiment of the apparatus according to the present invention being equipped with additional measurement functions but not connected to an EKG device,
Fig. 3a shows a curve of arterial pressure over time in the case of mechanical ventilation,
Fig. 3b shows a curve of arterial pressure over time in the case of spontaneous breathing,
Fig. 4a shows an EKG graph of a patient in a rhythmic condition, and
Fig. 4b shows an EKG graph of a patient with arrhythmia.

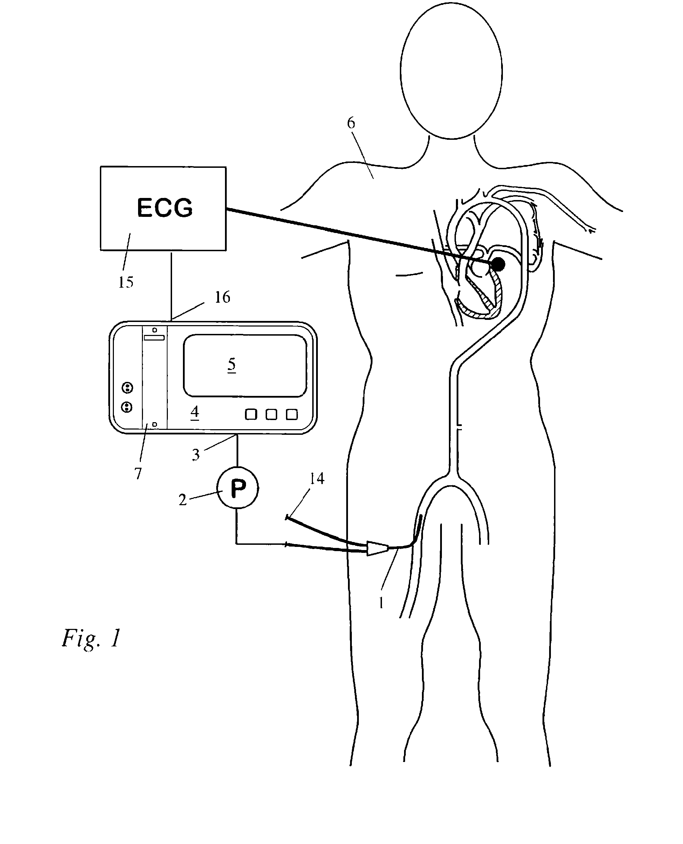

The apparatus of Fig. 1 comprises a patient monitor 4 reading in through an input channel 3 a pressure signal from a pressure sensor 2 measuring an arterial pressure of the patient 6 employing an arterial catheter 1. The patient monitor 4 includes storage means for storing a curve of arterial pressure over time P(t) determined from the read in pressure signal, an evaluation unit 7 programmed for determining physiologic parameters, such as cardiac output CO, from said pressure curve P(t) and a display 5 for displaying the physiologic parameters.

The sensor / catheter setup 1, 2 may be the same or similar as known from conventional patient monitoring systems, in particular patient monitoring systems employing pulse contour analysis methods such as commercially available under the trademark PiCCO. The additional catheter port 14 may be used for the withdrawl of blood samples, injection of pharmaceuticals or the like.

Though the sensor is shown to be invasive, a non-invasive pressure sensor may be implemented instead.

Through ECG input channel 16 the patient monitor 4 can also read in an electrocardiography signal from an ECG device 15.

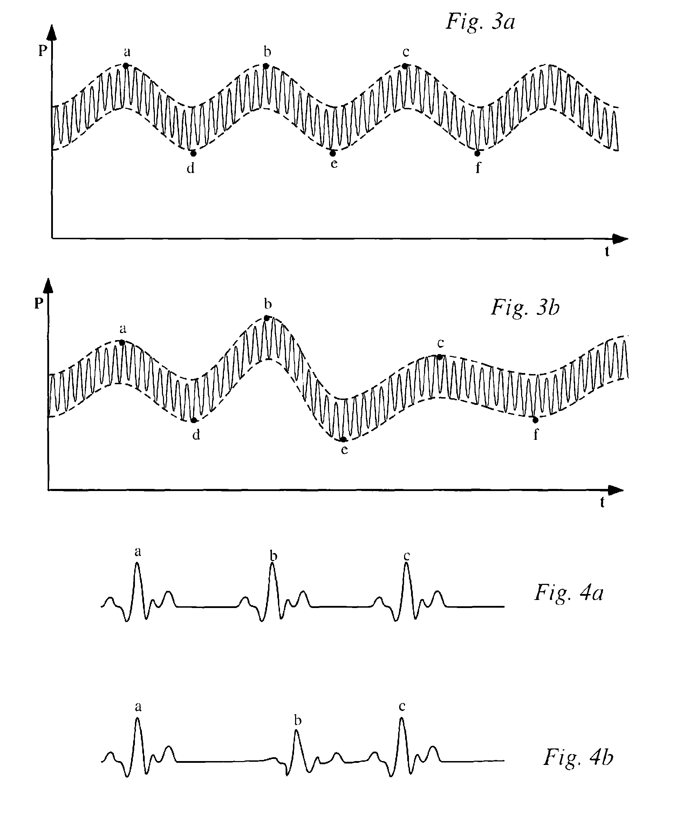

Depending on wether the patient 6 is mechanically ventilated or breathing spontaneously, the determined pressure curve P(t) will turn out differently as illustrated in Figs. 3a and 3b respectively. In case of mechanical ventilation, the envelope (indicated as a dashed line) of the arterial pressure curve P(t) will exhibit substantially equidistant intervals between its relative maxima a, b, c and/or minima c, d, e, respectively, as visible in Fig. 3a.

Therefore, if the condition ab = bc ... and/or de = ef ... and/or ab = bc = de = ef ... and/or ad = db = be = ce. is substantially met over a sufficient period of time (e.g. one minute) mechanical ventilation can be assumed from evaluating the pressure signal. '

Therein, ab, cd etc. may represent the actual distance distances between the extrema a and b, c and d etc., respectively, in the two dimensional coordinate system or a distance on the abscissa (i.e. the time interval between the two respective extrema). Usually, the latter alternative will be easier to implement.

Actually, the distances ab, be etc. will hardly ever be exactly equal taking into consideration that the precision of modern measurement equipment and electronic calculation is much higher than the mechanical and electrical precision of a ventilating machine, and that there will always be a slight variation of the mechanical response of the patient's 6 body to the externally imposed airway pressure.

Therefore, when determining from the pressure curve P(t) the patient's 6 breathing state, usually a certain variation between the distances ab, be etc. will be allowed to still indicate that the patient 6 is mechanically ventilated.

E.g. the distances ab and be can be considered substantially equal, if the difference ab - be is below a given suitable threshold value. Such a threshold value can be considered an extrema interval variation in the terminology of this application.

Therefore, the distances ab and bc etc. being substantially equal in the above sense means that a measure of variation of the distance between consecutive relative maxima and/or a measure of variation of a distance between consecutive relative minima is below a respective extrema interval variation.

If the patient is breathing spontaneously, the distances ab, bc, de, ef and the distances ad, db, be and cf will vary considerably as depicted in Fig. 3b.

Therefore, the variation of the distance between consecutive relative maxima and/or a measure of variation of a distance between consecutive relative minima will exceed a respective extrema interval variation in case of spontaneous breathing: The difference ab - bc will rise beyond a given suitable threshold value.

Threshold values of extrema interval variation can be determined empirically from sample arterial pressure curves of mechanically ventilated patients.

Similarly, the distances ab, bc etc. between consecutive R-peaks can be determined from read in electrocardiography signals.

Figs. 4a and 4b show a typical ECG graph of a patient in a rhythmic (Fig. 4a) or arrhythmic (Fig. 4b) state. If the distances between consecutive R-peaks are substantially equal, i.e. (using the terminology of this application) a measure of variation of the distance between consecutive R-peaks is below a respective RR interval variation threshold, the patient 6 is in a rhythmic state. If the distances between consecutive R-peaks are not substantially equal, i.e. (using the terminology of this application) a measure of variation of the distance between consecutive R-peaks exceeds a respective RR interval variation threshold, the patient 6 is in an arrhythmic state.

To enable a physician to assess volume responsiveness of the patient 6, the patient monitor 4 is adapted to determine, in a manner substantially known from conventional patient monitoring systems, the parameters PPV (Pulse Pressure Variation), SVV (stroke volume variation) and/or a volume responsiveness parameter derived from these parameters, and display such parameters on the display 5.

If, the distances ab, be etc. between consecutive R-peaks are substantially equal and if the distances ab, be, de, ef and/or ad, cb, be, ec, cf etc. of the envelope (Fig. 3a, 3b) are also substantially equal over a given period of time (e.g. one minute), the patient monitor 4 will actually display the parameters PPV, SVV and/or the volume responsiveness parameter on the display 5 in a certain display mode.

If, however, the distances ab, be etc. between consecutive R-peaks are substantially unequal or if the distances ab, be, de, ef and/or ad, cb, be, ec, cf etc. of the envelope (Fig. 3a, 3b) are substantially unequal, the patient monitor 4 will switch to a different display mode, i.e a different volume responsiveness indication state. Depending on the settings of the patient monitor 4 this different display mode includes that PPV, SVV and/or the volume responsiveness parameter are not displayed at all on the display. Instead or in addition, there may be displayed an indicator indicating that the patient 6 is breathing spontaneously (e.g. a text message "spontaneous breathing") and/or the patient 6 is in an arrhythmic state (e.g. a text message "arrhythmia").

Instead of the display mode depending on both criteria rhythmic/arrhythmic state and mechanical ventilation/spontaneous breathing, the invention can be carried out to make the display mode dependent on only one of these criteria.

Further, it is possible to implement different display modes for each of the cases arrhythmic state of a ventilated patient 6, rhythmic state of a ventilated patient 6, arrhythmic state of a spontaneously breathing patient 6 and rhythmic state of a ventilated patient 6.

The physician can thus be prevented from basing his decision about adding volume on parameters having been determined outside their actual regime of applicability.

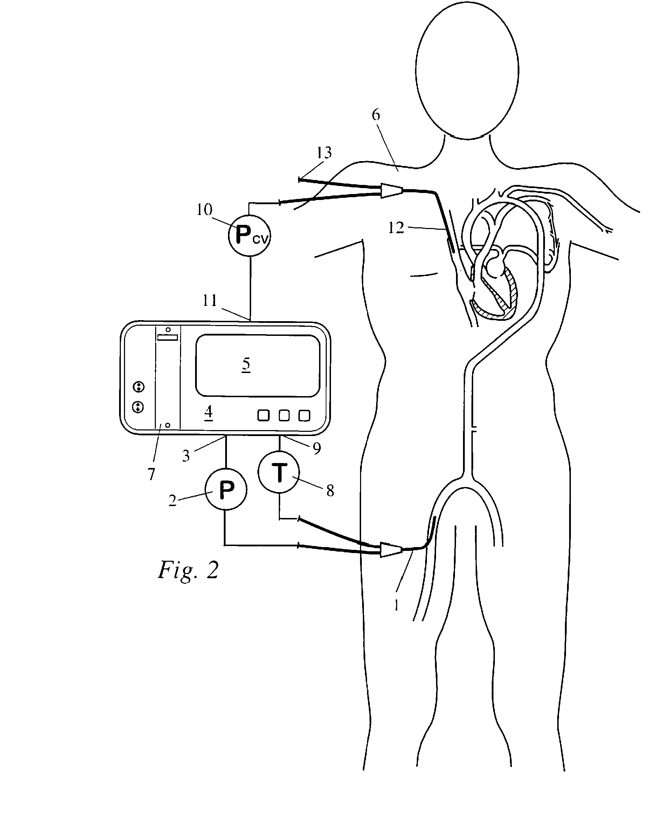

The apparatus of Fig. 2 comprises components corresponding to respective components illustrated in Fig. 1 and marked with corresponding reference numerals. In addition, the functionality of the apparatus is enhanced by a venous catheter setup and an arterial temperature measurement setup: The arterial catheter 1 is equipped with a temperature sensor 8 (such as a thermistor integrated near the catheter tip) to supply a temperature signal to the patient monitor 4 through another input channel 9. The temperature signal may serve for additional parameter determinations using thermodilution algorithms. For this purpose, a cold bolus can be injected through a respective port 13 of the central venous catheter 12. The thermodilution setup may advantageously be the same or similar as known from conventional patient monitoring systems employing thermodilution methods such as the above mentioned PiCCO. The another pressure signal may be read in through an additional input channel 3 which is acquired from a pressure sensor 10 measuring the central venous pressure of the patient employing the central venous catheter 12.

The apparatus of Fig. 2 is not connected to an ECG device. Instead, the display mode of the display 5 depends on curve characteristics of the recorded arterial pressure signal only.

To enable a physician to assess volume responsiveness of the patient 6, the patient monitor 4 is adapted to determine the parameters PPV, SVV and/or a volume responsiveness parameter derived from these parameters, and display such parameters on the display 5.

As explained above in connection with Figs. 1, 3a and 3b, the variation of the distance between consecutive relative maxima and/or a measure of variation of a distance between consecutive relative minima will exceed a respective extrema interval variation in case of spontaneous breathing: The difference ab - be will rise beyond a given suitable threshold value.

In this case, the patient monitor 4 switches to a display state in which the above parameters are not displayed and/or a warning indicator is displayed on the display 5.

If, the distances ab, be, de, ef and/or ad; cb, be, ec; cf etc. are substantially equal over a given period of time (e.g. one minute), the patient monitor 4 will switch back to a display mode, in which the parameters PPV, SVV and/or the volume responsiveness parameter are actually displayed on the display 5.

## Claims

1. Apparatus for determining physiologic parameters of a patient (6), said apparatus comprising
(a) at least one pressure sensor device (2) adapted to provide readings of a blood pressure of said patient (6),
(b) storage means for storing said readings as at least one pressure curve over time,
(c) an evaluation unit (4) for determining physiologic parameters from said pressure curve
**characterized**
**in that** said evaluation unit (4) is adapted to determine curve characteristics indicative for a breathing state and/or a heart rhythm state of said patient (6),
switch from a first volume responsiveness indication state to a second volume responsiveness indication state, if a first condition is met by said curve characteristics, and
to switch from said second volume responsiveness indication state to said first volume responsiveness indication state, if a second condition is met by said curve characteristics.

2. Apparatus according to claim 1, wherein said curve characteristics comprise the distance between consecutive relative maxima and/or consecutive relative minima of an envelope of said pressure curve,
said first condition includes that a measure of variation of said distance between consecutive relative maxima and/or a measure of variation of said distance between consecutive relative minima exceeds a respective first extrema interval variation threshold, and
said second condition includes that said measure of variation of said distance between consecutive relative maxima and/or said measure of variation of said distance between consecutive relative minima falls below a respective second extrema interval variation threshold.

3. Apparatus according to any one of the preceding claims, wherein said evaluation unit (4) further comprises an input channel for an electrocardiography signal,
said curve characteristics comprise the distance between consecutive R-peaks of said electrocardiography signal (RR-interval).
said first condition includes that a measure of variation of said distance between consecutive R-peaks exceeds a respective first RR interval variation threshold, and said second condition includes that said measure of variation of said distance between consecutive R-peaks falls below a respective second RR interval variation threshold.

4. Apparatus according to any one of the preceding claims, wherein said evaluation unit (4) is further adapted to determine a volume responsiveness indicator indicative for a volume responsiveness of said patient (6) and to output said volume responsiveness indicator.

5. Apparatus according to claim 4, wherein determination and/or output of said volume responsiveness indicator is disabled in said second volume responsiveness indication state, and
determination and output of said volume responsiveness indicator are both enabled in said first volume responsiveness indication state.

6. Apparatus according to claim 4 or 5, wherein said evaluation unit (4) is further adapted to determine at least one of pulse pressure variation (PPV) and stroke volume variation (SW) from said pressure curve, and
to determine said volume responsiveness indicator from at least one of said pulse pressure variation and stroke volume variation.

7. Apparatus according to any one of the preceding claims, wherein said evaluation unit (4) is further adapted to output an alert signal indicating whether the present volume responsiveness indication state is the first volume responsiveness indication state or the second volume responsiveness indication state.

8. Apparatus according to any one of the preceding claims, further comprising suppressing means for suppressing switching between said first volume responsiveness indication state and said second volume responsiveness indication state, wherein said suppressing means can be activated and de-activated by a user.

9. Method for determining physiologic parameters of a patient (6) using an electronic apparatus, said method including
- reading in a signal representing a blood pressure of said patient (6),
- storing said readings as at least one pressure curve over time,
- determining physiologic parameters from said pressure curve,
**characterised in that** said method further includes
determining curve characteristics indicative for a breathing state and/or a heart rhythm state of said patient (6),
switching said electronic apparatus from a first volume responsiveness indication state to a second volume responsiveness indication state, if a first condition is met by said curve characteristics, and
switching said electronic apparatus from said second volume responsiveness indication state to said first volume responsiveness indication state, if a second condition is met by said curve characteristics.

10. Method according to claim 9, wherein said curve characteristics comprise the distance between consecutive relative maxima and/or consecutive relative minima of an envelope of said pressure curve,
said first condition includes that a measure of variation of said distance between consecutive relative maxima and/or a measure of variation of said distance between consecutive relative minima exceeds a respective first extrema interval variation threshold, and
said second condition includes that said measure of variation of said distance between consecutive relative maxima and/or said measure of variation of said distance between consecutive relative minima falls below a respective second extrema interval variation threshold.

11. Method according to claim 9 or claim 10. wherein said method further includes the step of reading in an electrocardiography signal,
said curve characteristics comprise the distance between consecutive R-peaks of said electrocardiography signal (RR-interval),
said first condition includes that a measure of variation of said distance between consecutive R-peaks exceeds a respective first RR interval variation threshold, and said second condition includes that said measure of variation of said distance between consecutive R-peaks falls below a respective second RR interval variation threshold.

12. Method according to any one of claims 9-11, wherein said method further includes determining a volume responsiveness indicator indicative for a volume responsiveness of said patient (6) and outputting said volume responsiveness indicator.

13. Method according to claim 12, wherein said volume responsiveness indicator is disabled in said second volume responsiveness indication state, and
determination and output of said volume responsiveness indicator are both enabled in said first volume responsiveness indication state.

14. Method according to claim 12 or 13, wherein said method includes determining at least one of pulse pressure variation (PPV) and stroke volume variation (SVV) from said pressure curve. and
determining said volume responsiveness indicator from at least one of said pulse pressure variation and stroke volume variation.

15. Storage medium having physically stored thereon a computer program comprising executable instructions for causing a computer to perform a method according to any of claims 9-14.
